(19) Europäisches Patentamt
European Patent Office
Office européen des brevets

(11) **EP 4 446 988 A1**

(12) **EUROPEAN PATENT APPLICATION**

(43) Date of publication:
**16.10.2024 Bulletin 2024/42**

(21) Application number: **23167630.5**

(22) Date of filing: **12.04.2023**

(51) International Patent Classification (IPC):
***G06T 7/30*** (2017.01)

(52) Cooperative Patent Classification (CPC):
**G06T 7/30;** G06T 2207/10072; G06T 2207/20081;
G06T 2207/20084; G06T 2207/30004

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **TheraPanacea**
**75004 Paris (FR)**
• **Institut de Cancerologie Gustave Roussy**
**94805 Villejuif Cedex (FR)**
• **Centre Léon-Bérard**
**69373 Lyon Cedex 08 (FR)**

(72) Inventors:
• **LEROY, Amaury**
**75004 PARIS (FR)**
• **DEUTSCH, Eric**
**94805 VILLEJUIF (FR)**
• **GREGOIRE, Vincent**
**69373 LYON Cedex 08 (FR)**
• **PARAGIOS, Nikos**
**75004 PARIS (FR)**

(74) Representative: **Icosa**
**83 avenue Denfert-Rochereau
75014 Paris (FR)**

(54) **METHOD FOR GENERATING A REGISTERED IMAGE**

(57)     The present invention relates to a computer-implemented method for generating a registered image ($B_{reg}$) based on at least one first image of an object compliant with a source imaging modality and on at least one second image of said object compliant with a target imaging modality.

FIG. 2

**Description**

**FIELD OF INVENTION**

The present invention relates to the field of image synthesis and image processing, and more specifically to a method and a device for generating a registered image based on at least one first image of an object compliant with a source imaging modality and on at least one second image of said object compliant with a target imaging modality.

**BACKGROUND OF INVENTION**

Radiologic images like Magnetic Resonance Imaging (MRI) or Computed Tomography (CT) scans are the baseline source of information for all steps of cancer treatment. However, due to resolution constraints, data from MRI and CT scans are not accurate enough to provide in-depth and accurate assessment of tissue properties and of disease proliferation. It induces a high variability in tumor detection with a risk of toxic overdiagnosis or missed untreated areas. Whole-Mount-Histopathology (WMH) is considered gold standard by providing cell-level information on tumor environment from surgically resected specimens and can significantly improve radiologist's understanding of the links between tissue characteristics and radiologic images. However, a significant mismatch exists between tumor volumes outlined on radiologic images and bidimensional tumor representations on WMH slides.

An accurate co-registration between high-resolution digitized WMH and radiologic 3D images would address the aforementioned issues.

**SUMMARY**

This invention thus relates to a computer-implemented method for generating a registered image based on at least one first image of an object compliant with a source imaging modality and on at least one second image of said object compliant with a target imaging modality, the method comprising:

- receiving said at least one first image and said at least one second image,
- transforming said at least one first image into at least one first transformed image, so that said at least one first transformed image is compliant with said target imaging modality, by using a first machine learning model,
- implementing a second machine learning model receiving as inputs said at least one second image and at least one first transformed image, so as to obtain at least one first registered image,
- implementing a third machine learning model, said third machine learning model receiving as inputs said at least one first transformed image and said at least one first registered image, so as to obtain said registered image, wherein said registered image is registered on said at least one first transformed image.

Thanks to the invention, it is possible to register two images generated by two different imaging modalities that have different imaging properties. In this manner, characteristics of the object represented on both images can be mutualized. In the context of clinical diagnosis, this property is of huge interest to enhance the diagnosis by using a plurality of inputs and being able to perform analysis based on those inputs in a concomitant manner. The registration is advantageously decomposed into a coarse registration step followed by a fine registration step.

Advantageously, the object is a human body part. For instance, the object is a part of the head and neck of a human body. In other examples, the object is the prostate or the pelvic area of a person.

In some embodiments, the at least one first image comprises at least one 2D image and wherein the at least one second image comprises one 3D image, said at least one 3D image comprising a plurality of N 2D sections of the object. In those embodiments, the source imaging modality outputs 2D images and the target imaging modality outputs 3D images. The invention allows registering the at least second image, which is a 3D image, with the at least first image, which is a 2D image.

In some other embodiments, the at least one first image comprises at least one 3D image comprising a plurality of N 2D sections of said object and wherein the at least one second image comprises at least one 2D image. In those embodiments, the source imaging modality outputs 3D images and the target imaging modality outputs 2D images. The invention allows registering the at least second image, which is a 2D image, with the at least first image, which is a 3D image.

Therefore, the method according to the invention can be implemented for both registration of 2D images with 3D images and registration of 3D images with 2D images.

In some embodiments, implementing said second machine learning model comprises iterations, each iteration among said iterations comprising:

- i) registering the at least one second image onto said at least one first transformed image by applying a current global rigid transform to the at least one second image, to obtain at least one current second transformed image, said current second transformed image comprising N current 2D transformed sections of the object,
- ii) matching each first transformed image among the at least one first transformed images to one corresponding current 2D transformed section among the N current second transformed sections so as to meet a similarity criterion, the similarity criterion being evaluated on the basis of a similarity measure estimated between each first transformed image and one corresponding current 2D transformed section.

It is possible that the at least one first image comprises less images than the at least one second image. Therefore, step ii) aims at finding a match between each image comprised in the at least one first image and one corresponding image among the at least one second image. Step ii) of each iteration thus increases the accuracy of the coarse registration step.

In some embodiments the method further comprises, preliminarily to implementing the second machine learning model:

- obtaining at least one first segmentation mask corresponding to structures of interest in said at least one first image,
- obtaining at least one second segmentation mask corresponding to said structures of interest in said plurality of N 2D sections,

step i) of each iteration comprising implementing said second machine learning model receiving as additional inputs said at least one first segmentation mask and said at least one second segmentation mask, so as to obtain said current global rigid transform.

When the object is a human body part, advantageously, the structures of interest are stiff regions such as bones, cartilage, or tendons.

Therefore, when the object is a human body part, the method according to the invention advantageously leverages the presence of stiff regions in the human body part to guide the registration. This is advantageous when, for instance, the source imaging modality is histology (or whole slide imaging). Indeed, stiff regions are less affected, i.e. less deformed, by the process of putting the sample on a slide; therefore, those regions constitute interesting landmarks to serve as reference in a registration process.

In some embodiments, step ii) of each iteration comprises updating, for each first transformed image, a coordinate along a common axis of said plurality of N current 2D transformed sections, by maximizing the similarity measure. As explained before, step ii) aims at finding a match between each image comprised in the at least one first image and one corresponding image among the at least one second image. Step ii) of each iteration increases the accuracy of the coarse registration step. This can be of use for example when the target imaging modality is computed tomography imaging, as often, the at least one second image comprises 2D sections recorded along an axis recorded.

Advantageously, the first machine learning model is a cycle generative adversarial network, GAN, said first machine learning model being configured to generate, from an input source image compliant with said source imaging modality, a simulated image compliant with said target imaging modality respectively associated with said input source image.

In other embodiments, the first machine learning model can be any modality translation architecture like diffusion or autoencoder models.

The step of translating the at least one first image into images compliant with the target imaging modality further help the registration process. Indeed, such a registration process is difficult when the images to be registered present different visual characteristics. For instance, a histological image is colored and presents a resolution in the order of 0.5 microns, while a 3D CT-scan image is in black and white and presets a resolution in the order of millimeters, which renders the comparison of those kinds of images difficult. Therefore, translating the at least one first image into images compliant with the target imaging modality makes comparison steps for the registration process easier as comparable images are compared. The Cycle-GAN architecture advantageously allows implementing a style transfer task and is thus well-suited for the step of translation.

Advantageously:

- said at least one first transformed image corresponds to a first 3D representation of the object,
- the at least one first registered image comprises one 3D first registered image with voxels and
- the third machine learning model outputs, for each voxel, displacement information corresponding to a displacement between said one 3D first registered image and the first 3D representation.

Implementing the third machine learning model allows thus refining the registration obtained with the second model, on a voxel basis.

Advantageously, for each voxel, the displacement information takes into account a regularization parameter that is a function of the voxel position with respect to the structures of interest.

Indeed, when the object is a human body part, and when, for instance, the source imaging modality is histology (or whole slide imaging), such a regularization parameter help account for manipulations specific to the histological process. Soft tissues away from bones and cartilage are subject to shrinkage or disruption as nothing holds them, while tissues close to stiff areas are less likely to undergo severe deformation. With the regularization parameter, the displacement information may be controlled depending on the distance to stiff areas, with close tissue being more highly constrained than isolated areas.

Advantageously, the source imaging modality is histopathology, whole slide imaging, echography, or radiography.

Advantageously, the target imaging source modality is computed tomography imaging or magnetic resonance imaging.

Another aspect of the invention relates to a device comprising a processor configured to carry out the method previously described.

Another aspect of the invention relates to a computer program product comprising instructions, which, when the program is executed by a computer, cause the computer to carry out the method previously described.

## DEFINITIONS

In the present invention, the following terms have the following meanings:

**"Image registration"** refers to the process of transforming an input image to align with a target image into one coordinate system. A reference frame (i.e.; referential) associated to the target image is stationary, while the input image is transformed to match to the target image.

**"Human body part"** refers to a part of a human body, for example:

- a member (e.g., head, trunk, arm, leg...);
- a system (e.g., cardiovascular, circulatory, digestive, endocrine, locomotor, nervous, reproductive, respiratory, sensory, urinary...);
- an organ (e.g., brain, heart, spleen, stomach, intestines, colon, bladder, pancreas, kidney, liver, lungs, thyroid, genitals...); or
- a tissue (muscle, nervous, connective, epithelial...).

By **"structures of interest",** it is meant any area of interest that can guide a registration process, such as a coarse registration step as will be seen further. For instance, when considering a human body part, structures of interest may be stiff structures like cartilage, tendons, bones that are supposedly not moving because during a histological process, unlike soft tissues that undergo multiple deformations. In other cases pertaining to human body parts without bones like prostate and bladder, structures of interest may be constituted by portions of the human body part under examination.

By **"2D section of a 3D representation",** it is meant a 2D representation of a section (or a "slice") of the 3D representation. For example, the 3D representation may comprise a voxel grid along 3 axes (x,y,z). A 3D section of the 3D representation may correspond to the subset of voxels (x,y) when one of the coordinate (z) is fixed. The corresponding 2D section may then be defined as the 2D projection of the 3D section on a plane (x,y). For example, a respective pixel (x,y) may be associated with each voxel (x,y) of the 3D section and its value may be set to the value of the corresponding voxel.

In the context of CT scan, the sections may be advantageously cross sections, but the sections may be performed along any axis (longitudinal, sagittal, or other random axis orientation of interest).

The term **"processor"** should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processorreadable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

## BRIEF DESCRIPTION OF THE DRAWINGS

**Figure 1** illustrates a device for generating a registered image based on at least one first image of an object compliant with a source imaging modality and on at least one second image of said object compliant with a target imaging modality according to some embodiments.

**Figure 2** is an example of a flow-chart representing a method for generating a registered image based on at least one first image of an object compliant with a source imaging modality and on at least one second image of the object compliant with a target imaging modality.

**Figure 3** represents an example of a machine learning architecture for registering at least one second image onto at least one first image based on a rigid transform.

**Figure 4** represents an example of a machine learning architecture for matching first transformed images to corresponding 2D sections of a second image.

**Figure 5** represents an example of a machine learning architecture for refining a registration between two images, where the registration is obtained by the examples of machine learning architecture of Figure 3 and Figure 4.

**Figure 6** shows results obtained with the method for generating a finely registered image based on at least one first image of an object compliant with a source imaging modality and on at least one second image of the object compliant with a target imaging modality according to embodiments of the invention.

**Figure 7** shows other results obtained with the method for generating a registered image based on at least one first image of an object compliant with a source imaging modality and on at least one second image of the object compliant with a target imaging modality according to embodiments of the invention.

## DETAILED DESCRIPTION

This invention relates to a method 100 for generating a registered image based on at least one first image of an object compliant with a source imaging modality and on at least one second image of said object compliant with a target imaging modality.

The object is for instance a part of a human body under medical examination.

In what follows, the at least one first image comprises at least one 2D image and the at least one second image comprises one 3D image. The 3D image typically comprises a plurality of N 2D sections, N being an integer higher or equal to 1. However, the method 100 can be implemented when the at least one first image comprises one 3D image and the at least one second image comprises at least one 2D image.

For instance, the at least one first image is compliant with a source imaging modality chosen among histopathology, whole slide imaging, or echography. For instance, the at least one second image is compliant with a target imaging modality chosen among computed tomography imaging or magnetic resonance imaging.

One of the goals achieved by the method 100 is to register the at least one 2D image with the 3D image.

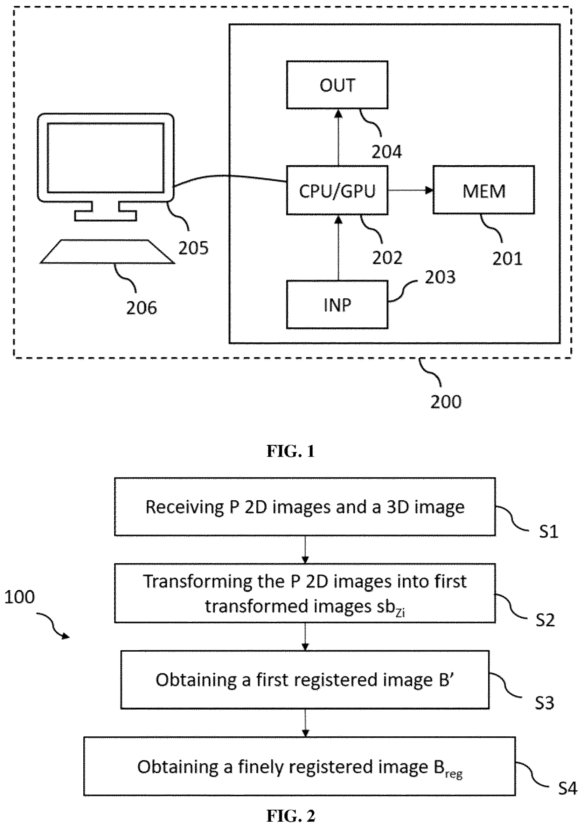

The method 100 may be implemented with a device 200 such as illustrated on **Figure 1.**

**Figure 1** illustrates a device 200 for generating a registered image based on at least one first image of an object compliant with a source imaging modality and on at least one second image of said object compliant with a target imaging modality according to embodiments of the invention.

In these embodiments, the device 200 comprises a computer, this computer comprising a memory 201 to store program instructions loadable into a circuit and adapted to cause a circuit 202 to carry out steps of the method of **Figure 2** when the program instructions are run by the circuit 202. The memory 201 may also store data and useful information for carrying steps of the present invention as described above.

The circuit 202 may be for instance:

- a processor or a processing unit adapted to interpret instructions in a computer language, the processor or the processing unit may comprise, may be associated with or be attached to a memory comprising the instructions, or

- the association of a processor / processing unit and a memory, the processor or the processing unit adapted to interpret instructions in a computer language, the memory comprising said instructions, or

- an electronic card wherein the steps of the invention are described within silicon, or

- a programmable electronic chip such as a FPGA chip (for « Field-Programmable Gate Array »).

The computer may also comprise an input interface 203 for the reception of input data and an output interface 204 to provide output data. Examples of input data and output data will be provided further.

To ease the interaction with the computer, a screen 205 and a keyboard 206 may be provided and connected to the computer circuit 202.

**Figure 2** is a flowchart illustrating an example of steps to be executed to implement the computer-implemented method 100. For simplicity, it is considered that the at least one first image comprises P 2D images $a_{Zi}$, with $Z_j$ an index, P an integer higher or equal to 1, and that the at least one second image consists in one 3D image B.

In a step S1, the P 2D images $a_{Zi}$ and the 3D image B are received by the input interface 203 of the device 200. The P 2D images $a_{Zi}$ comprise a representation of an object and the 3D image B comprises a 3D representation of the same object. The 3D image B is composed of a sequence of N 2D consecutive sections $b_j$ of the object. The N 2D consecutive sections $b_j$ are taken along an axis. Typically, N is higher than P. For example, N is five times higher than P.

For instance, the P 2D images $a_{Zi}$ are compliant with a source imaging modality chosen among histopathology, whole slide imaging, or echography. For instance, the 3D image B is compliant with a target imaging modality chosen among computed tomography imaging or magnetic resonance imaging.

In a step S2, the P 2D images $a_{Zi}$ are transformed into P so-called first transformed images $sb_{Zi}$ by using a first machine learning model. Step S2 is for instance implemented by the circuit 202 of the device 200. The P first transformed images $sb_{Zi}$ are simulated target images compliant with the target imaging modality. By simulated target image, it is meant an image that "looks like" an image obtained with the target imaging modality but that was not obtained in reality with the target imaging modality and underwent processing in order to "look like" an image obtained with the target imaging modality. The reference $sbz_i$ is meant to indicate that the P first transformed images are synthetic, or simulated, images looking like images that would have been obtained with the target imaging modality.

In a step S3, a first registered image B' is obtained by implementing a second machine learning model. In that goal, the second machine learning model receives as inputs the P first transformed images $sb_{Zi}$ and the 3D image B. The P first transformed images $sb_{Zi}$ form together a 3D stack sB. The obtained first registered image B' is then coarsely registered with the 3D stack sB.

Finally, in a step S4, a registered image $B_{reg}$ is obtained by implementing a third machine learning model. In that goal, the third machine learning model receives as inputs the first registered image B' and the P first transformed images $sB_{Zi}$. The obtained registered image $B_{reg}$ is then finely registered with the 3D stack sB. In other words, the registration - or alignment, of the registered image $B_{reg}$ with the 3D stack sB is improved as compared to the registration of the first registered image B' with the 3D stack sB obtained at the end of step S3.

Now, steps S2, S3 and S4 will be described more in details.

The first machine learning model generates, from one image corresponding to a given imaging modality, a corresponding image that "synthetizes" the image that would have been obtained of the same object by using another imaging acquisition principle than the one that have been used. In other words, the overall appearance (including edges) is the same between the input image and the simulated image, but the simulated image "looks like" an image obtained by the other imaging modality, while not having been acquired by this other imaging modality.

Advantageously, the first machine learning model is a Cycle-GAN.

A Cycle-GAN consists of two generators $G_{A->B}$ and $G_{B->A}$ and two discriminators $D_A$ and $D_B$. The generators $G_{A->B}$ and $G_{B->H}$ are trained to convert images from one imaging modality to another, while the discriminators $D_A$ and $D_B$ aim to distinguish between real and fake, or simulated, images. The cycle consistency loss is used to ensure that the reconstructed image from the other domain is close to the original input image, and the adversarial loss encourages the generators $G_{A->B}$ and $G_{B->A}$ to produce realistic images, that is, images resembling images compliant to the input imaging modality of the corresponding generator. The training process involves alternating between training the generators $G_{A->B}$ and $G_{B->A}$ and discriminators $D_A$ and $D_B$, optimizing their respective losses until the generators $G_{A->B}$ and $G_{B->A}$ are able to produce high-quality, realistic images in both directions.

For instance, the generators $G_{A->B}$ and $G_{B->A}$ of the Cycle-GAN are trained to convert images from the source imaging modality to the target imaging modality and reversely.

For instance, the generators $G_{A->B}$ and $G_{B->A}$ are trained with a loss function comprising several terms.

A first term $L_{adv}$ is linked to the adversarial training related to the Cycle-GAN. Indeed, the Cycle-GAN comprises two neural networks fighting against each other and improving themselves to fight better. An example for the first term $L_{adv}$ is the sum of two components $L_{adv}(a)$ and $L_{adv}(b)$, the first component being related to the target imaging modality and of the form:

$$L_{adv}(b) = \log\big(D_B(b)\big) + \log\big(1 - D_B \circ G_{A \to B}(a)\big)$$

where a denotes a training image obtained with the source imaging modality and b denotes a training image obtained with the target imaging modality. The second component is the symmetric term of $L_{adv}(b)$, that is, $L_{adv}(a)$, related to the source imaging modality, where $D_B$ is replaced by $D_A$ and $G_{A\text{-}>B}$ is replaced by $G_{B\text{-}>A}$, a is replaced by b and b is replaced by a.

A second term $L_{ID}$ is meant to encourage modality specific feature representation when considering one of the P 2D images (respectively one of the N 2D sections) being the input for $G_{A\text{-}>B}$ ($G_{B\text{-}>A}$ respectively) with an expected identity synthesis. An example for the second term $L_{ID}$ is the sum of two components $L_{ID}(a)$ and $L_{ID}(b)$, the first component being related to the target imaging modality and of the form:

$$L_{ID}(b) = \|G_{A\to B}(b) - b\|_1.$$

where b denotes a training image obtained with the target imaging modality. The second component is the symmetric term of $L_{ID}(b)$, that is, $L_{ID}(a)$, related to the source imaging modality, where $G_{A\text{-}>B}$ is replaced by $G_{B\text{-}>A}$ and b is replaced by a, a denoting a training image obtained with the source imaging modality.

A third term $L_{MIND}$ is relating to a modality-independent neighbourhood descriptor (MIND) and is meant to ensure style transfer without content alteration by examining the local structure similarity around a sliding patch. An example for the third term $L_{MIND}$ is the sum of two components $L_{MIND}(a)$ and $L_{MIND}(b)$, the first component being related to the source imaging modality and of the form:

$$L_{MIND}(a) = \frac{1}{9}\left\|MIND_x\big(G_{A\to B}(a)\big) - MIND_x(a)\right\|_1.$$

where a denotes a training image obtained with the source imaging modality. The second component is the symmetric term of $L_{MIND}(a)$, that is, $L_{MIND}(b)$, related to the target imaging modality, where $G_{A\text{-}>B}$ is replaced by $G_{B\text{-}>A}$ and a is replaced by b, b denoting a training image obtained with the target imaging modality.

A fourth term $L_{cyc}$ is a pixel-wise cycle loss term relating to the cyclical pattern lying in the similarity between the original images and the reconstructed images. An example for the fourth term $L_{cyc}$ is the sum of two components $L_{cyc}(a)$ and $L_{cyc}(b)$, the first component being related to the source imaging modality and of the form:

$$L_{cyc}(a) = \|G_{B\to A} \circ G_{A\to B}(a) - a\|_1.$$

where a denotes a training image obtained with the source imaging modality. The second component is the symmetric term of $L_{cyc}(a)$, that is, $L_{cyc}(b)$, related to the targe imaging modality, where $G_{A\text{-}>B}$ is replaced by $G_{B\text{-}>A}$, $G_{B\text{-}>A}$ is replaced by $G_{A\text{-}>B}$ and a is replaced by b, b denoting a training image obtained with the target imaging modality.

The global loss function is a weighted sum of all those four terms.

Advantageously, the first machine learning model is trained preliminary to the implementation of the method 100 with training data comprising pairs of images, both images from each pair of images being obtained, one from the source imaging modality, the other from the target imaging modality and representing similar objects. For instance, when the object is a human body part, both images of a pair of images represent the same human body part. Advantageously, the training data comprises pairs of images from a plurality of human body parts of a plurality of patients. A pair of images may comprise images from different patients.

Thus, in some embodiments, in step S2, the P 2D images $a_{Zi}$ are transformed into the P first transformed images $sb_{Zi}$ by using the first machine learning model (once this one has been trained). The P 2D images $a_{Zi}$ are compliant with the source imaging modality. The P first transformed images $sb_{Zi}$ are, as explained before, simulated images compliant with the target imaging modality.

Now step S3 of the method 100 will be described more in details.

In step S3, a second machine learning model is implemented by carrying out a plurality of iterations. The input data received by the second machine learning model comprise the 3D image B and the P first transformed images $sb_{Zi}$, which form together the 3D stack sB.

When implementing the second machine learning model, each iteration comprises:

- i) registering the 3D image B with the 3D stack sB by applying a current global rigid transform to the 3D image B, to obtain one so called current second transformed image. The current second transformed image is a 3D image comprising N current 2D transformed sections $b'_i$ of the object; each among the N current 2D transformed sections bji corresponds to a version of one 2D section among the N 2D sections of the object, obtained by application of the current global rigid transform;
- ii) matching each first transformed image $sb_{Zi}$ among the P first transformed images $sb_{Zi}$ to one corresponding current 2D transformed section among the N current second transformed sections so as to meet a similarity criterion. The similarity criterion is evaluated on the basis of a similarity measure estimated between each first transformed image $sb_{Zi}$ and one corresponding current 2D transformed section.

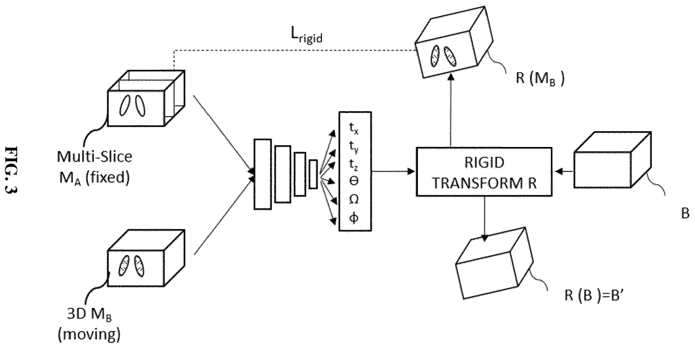

In some embodiments illustrated on **Figure 3,** step i) of each iteration comprises obtaining a first 3D segmentation mask $M_A$ corresponding to structures of interest in the 3D stack sB, and obtaining a second 3D segmentation mask $M_B$ corresponding to the same structures of interest in the current second transformed image. The second 3D segmentation mask $M_B$ can be obtained by well-known segmentation techniques such as manual contouring by health practitioners, thresholding, or implementation of machine learning models.

For instance, the first 3D segmentation mask $M_A$ can be obtained from P so called first 2D segmentation masks. The P first 2D segmentation masks can be obtained from the P 2D images $a_{Zi}$. Then, the P first 2D segmentation masks are completed with void masks, so as to, after aggregation, form the first 3D segmentation mask $M_A$. The P first 2D segmentation masks can be obtained by well-known segmentation techniques, such as manual contouring, thresholding, or implementation of machine learning models.

When the object is a part of a human body, the structures of interest can be stiff structures, such as bones, cartilage or tendons. When the source imaging modality is one among histopathology or whole slide imaging, this kind of structures of interest is advantageously leveraged in a registration procedure, as they are less affected, deformed or damaged than soft structures. They can therefore be used as reference landmarks.

N second 2D segmentation masks are obtained by extracting from the second 3D segmentation mask 2D segmentation masks $M_{Bzi}$ in planes corresponding to the N current 2D transformed sections $b'_i$.

In some embodiments, step ii) of each iteration comprises updating, for each first transformed image $sb_{Zi}$, a coordinate along a common axis of the plurality of N current 2D transformed sections $b'_i$, by maximizing a similarity measure. For instance, when the second imaging modality is CT scan, the N current 2D transformed sections b'; may be cross sections along any axis (longitudinal, sagittal, or other random axis orientation of interest).

More specifically, in step ii), assuming that the N current 2D transformed sections b; correspond to sections along a common axis and having a coordinate i along this axis, for each first transformed image $sb_{Zi}$, a match with one among the N current 2D transformed sections $b'_i$ is searched for, and a coordinate Zi is assigned to each of the first transformed images $sB_{Zi}$.

As illustrated on **Figure 3**, the first 3D segmentation mask $M_A$ and the second 3D segmentation mask $M_B$ are used to train the second machine learning model. The second machine learning model is for instance an encoder followed by a fully connected layer that outputs six transformation parameters (three rotation angles, $\Theta$, $\Omega$, $\Phi$, three translation amounts $t_x$, $t_y$, $t_z$) representative of a rigid transformation $R(M_A, M_B)$ that wraps the current second transformed image onto the 3D stack. The six transformation parameters define a spatial transform R. For instance, the second machine learning model can be trained by using a loss function $L_{rigid}$ defined by:

$$L_{rigid}\left(M_A, R(M_A, M_B)\right) = \sum_{i=1}^{P} DSC\left(M_{Azi}, R(M_A, M_B)(M_{Bzi})\right)$$

where $M_{Azi}$ denotes the first 2D segmentation mask in the plane corresponding to the plane of coordinate $Z_i$ and to one first transformed image $sb_{zi}$ among the P first transformed images,

$M_{Bzi}$ denotes the second 2D segmentation mask in the plane corresponding to the plane of coordinate Zi and to the current 2D section b'; associated to the first transformed image $sb_{Zi}$,

$R(M_A, M_B) (M_{Bzi})$ denotes the result of the application of the rigid transform to the current 2D section b'; associated to the first transformed image $sb_{Zi}$, DSC denotes the Dice Similarity Coefficient.

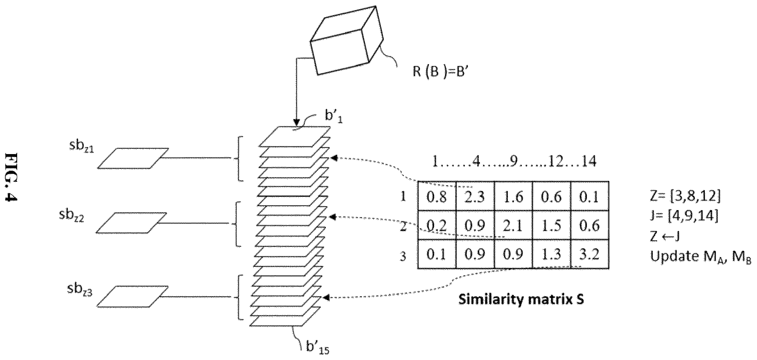

In some embodiments illustrated on **Figure 4,** step ii) is performed by solving a sequence alignment problem. Sequence alignment originates from bioinformatics and consists in a problem where two or more DNA, RNA, or protein sequences are compared to identify similarities and differences. The goal is to find the best possible alignment between the sequences by inserting gaps and mismatches as necessary, in order to infer evolutionary relationships and functional similarities.

Here, the problem which is solved is to find, for each among the P first transformed images $sb_{zi}$, one corresponding 2D current transformed section among the N current 2D transformed sections. A similarity matrix S is built, comprising terms S(i,j), for i and integer comprised between 1 and P and j an integer comprised between 1 and N defined by:

$$S(i,j) = \begin{cases} MI\left(sb_{Z_i}, b_{J_j}\right) & if\ i = 1 \\ max_k\left(S(i-1,k) + MI\left(sb_{Z_i}, b_{J_j}\right)\right) & otherwise \end{cases}.$$

where MI denotes the Mutual Information metrics,

$sb_{zi}$ denotes the first transformed image corresponding to the plane of coordinate $Z_i$,

$b_{Jj}$ denotes the current 2D section corresponding to the plane of coordinate $J_j$.

In other words, each row of S will be filled by computing the sum of the mutual information for the corresponding column j and the maximum similarity from the last row.

The building of the similarity matrix S is dynamic, and outputs, at the end of step ii), a sequence of matches between each of the P first transformed images $sb_{zi}$ and one corresponding 2D current transformed section $b_{Jj}$ among the N current 2D transformed sections $b'_{Jj}$. In other words, one sequence J=[$J_1$,...$J_P$] is found at each iteration of step ii). An initial sequence Z= [$Z_1$,...$Z_P$] is then updated by taking the values of the found sequence J=[$J_1$,...$J_P$]. The sequence J found at the end of each iteration of step ii) can be viewed as an update of the best set of 2D current transformed sections $b'_{Jj}$ among the N current 2D transformed sections $b'_j$ to match the P first transformed images $sb_{zi}$.

The combination of step i) and step ii) is repeated a given number of times.

The number of iterations is defined to reach a good balance between computational time and similarity maximization. Typically, a number of three iterations is implemented and provides a good compromise between the registration accuracy and the computational time.

After the training of the second machine learning model, the spatial transform R is applied to the 3D image B so as to obtain the first registered image B' equal to R(B).

Therefore, at the end of step S3, the first registered 3D image B' is obtained. This first registered 3D image B' is coarsely registered with the 3D stack sB formed by the P first transformed images $sb_{zi}$.

Now step S4 will be described more in detailed. Step S4 aims at refining the registration between the first registered 3D image B' and the 3D stack sB, by transforming the first registered 3D image B' into a second registered 3D image $B_{reg}$. In other words, the second registered 3D image $B_{reg}$ will be better, i.e. more accurately, registered with the 3D stack sB than the coarsely registered 3D image B' is.

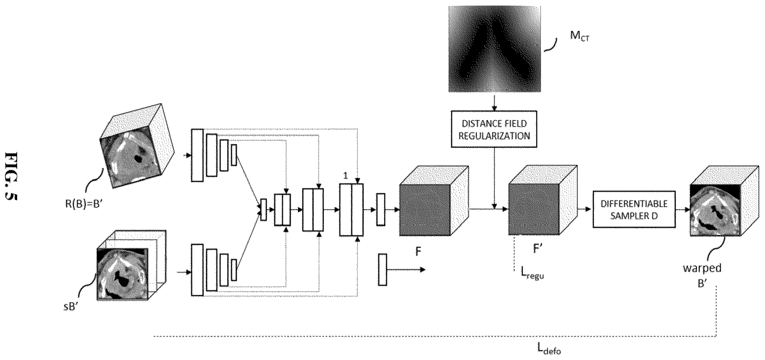

In some embodiments illustrated on **Figure 5**, step S4 comprises implementing a third machine learning model. The third machine learning model will learn a non-linear mapping between the first registered image B' and the 3D stack sB. The learned non-linear mapping will then be used to deform the first registered 3D image B' into the registered image $B_{reg}$. The registered image $B_{reg}$ will then be finely registered with the 3D stack sB.

The first registered 3D image B' comprises N*R*S voxels, where RxS is the common size of the N current 2D transformed sections. In order for the third machine learning model to receive two 3D images of the same size, the 3D stack sB, which contains the P first transformed images $sb_{zi}$, is complemented with empty voxels (i.e. with an intensity value equal to zero), so as to obtain a so-called completed 3D stack $sB_{complete}$ with N*R*S voxels.

As illustrated on **Figure 5**, the third machine learning model receives as inputs the first registered 3D image B' and the completed 3D stack $sB_{complete}$. For instance, the third machine learning model comprises two parallel encoders to which a decoder is connected. The third machine learning model outputs a mapping in the form of a displacement field F. The displacement field F has the size of the first registered 3D image B' and of the completed 3D stack $sB_{complete}$. Three displacement values, for each direction, are associated to each voxel of the displacement field F. A differentiable sampler D corresponding to the displacement field F then warps the coarsely registered 3D image B' onto the completed 3D stack $sB_{complete}$.

For instance, the third machine learning model is trained with a loss function that measures the difference between the transformed image obtained with the third machine learning model and a target image.

By means of example, a loss function $L_{defo}$ can be defined as follows:

$$L_{defo}\left(sB_{complete}, D\left(sB_{complete}, B'\right)\right) = \sum_{i=1}^{P} NCC\left(sb'_{Z_i}, D(sB', B')\left(b'_{Z_i}\right)\right),$$

where NCC denotes a normalized cross correlation metrics,

$sb'_{Zi}$ denotes the first transformed image corresponding to the coordinate $Z_i$ in the completed 3D stack $sB_{complete}$,

$b'_{Zi}$ denotes the current 2D section of coordinates $Z_i$ in the coarsely registered 3D image B' and corresponding to the first transformed image $sb'_{Zi}$.

Advantageously, when the source imaging modality is histology, a regularization parameter is added to the displacement field F. The regularization parameter is determined based, for each voxel of the displacement field F, on the position of the voxel to the structures of interest determined during the substeps i) of the iterations of step S3. Indeed, it is assumed here that soft tissues away from bones and cartilage are subject to shrinkage or disruption as nothing holds them, while tissues close to stiff areas are less likely to undergo severe deformation. Therefore, the regularization parameter is defined such that areas close to those stiff structures are more highly constrained than other areas that are more isolated.

By way of example, a distance transform map $\Delta$ can be generated from the second 3D segmentation mask $M_B$ obtained in step S3 and based on an Euclidean metric, and defined by:

$\Delta(v) = min_{m \in M_{ct}} \|v - m\|_2$. This distance transform map maps each voxel $v$ of the 3D image B to its distance with the closest point $m$ to the structures of interest present in the second 3D segmentation mask $M_B$. An effective displacement field F' taking into account the above-mentioned regularization parameter can be defined by:

$$F' = F \odot (\Delta + \varepsilon),$$

where $\odot$ denotes the Hadamard product,

$\varepsilon$ denotes a hyperparameter matrix allowing small displacement even for cartilage areas (i.e. stiff areas) for which the distance transform $\Delta$ is null.

Advantageously, another regularization parameter can be included that is meant to encourage smooth deformation. This other regularization parameter may advantageously be used with the previous regularization parameter relating to closeness to stiff areas. The loss function, for training the third machine learning model, may then include an additional term $L_{regu}$ defined, for instance, by:

$$L_{regu}(F') = \sum_{v \in R^3} \|\nabla F'(v)\|_2,$$

where $\nabla$ denotes the gradient operator,

the condition $v \in R^3$ means that the whole volume is constrained by the application of the gradient operator.

Advantageously, the first machine learning model, the second machine learning model, and the third machine learning model are trained end-to-end, so that each model benefits from the signal of one another.

**Experiments and results**

Performances of the method 100 for generating a registered image based on at least one first image of an object compliant with a source imaging modality and on at least one second image of said object compliant with a target imaging modality have been assessed with a clinical dataset relating to 108 patients on whom both a pre-operative CT scan and 4 to 11 WSIs after laryngectomy were acquired. For clinical validation of the method 100, expert radiation oncologists delineated the Gross Tumor Volume (GTV) on CT, while expert pathologists contoured the tumor on WSIs. The thyroid and cricoid cartilages were segmented using a commercial software. CT 3D images of size 256x256x64 of 1mm isotropic grid space were obtained. The dataset was split patientwise into three groups for training (64), validation (20), and testing (24).

The first machine learning model consisted in a Cycle-GAN comprising two generators with a U-Net architecture and two discriminators with a PatchGAN architecture with the addition of residual blocks between the contracting and expanding paths.

The second machine learning model and the third machine learning model also adopt the U-Net architecture, but the second machine learning model (rigid module) only makes use of the encoding block, while the third machine learning model (deformable module) is re-implemented with two encoding heads merged by subtraction and one decoder.

The weight of the regularization $L_{regu}$ is 0.01. The part of the architecture for the second machine learning model dedicated to step ii) in the iterations of step S3 was implemented with three consecutive dual blocks, as it was the best balance between the convergence of the objective function and the runtime. Adam optimizers were systematically selected for gradient descent calculus, with $\beta 1 = 0.5$, $\beta 2 = 0.99$. The learning rates were set to $2 \times 10^{-4}$, $1 \times 10^{-3}$, $1 \times 10^{-4}$ for the three different blocks with a linear decrease after half of training.

The first machine learning model, the second learning machine learning model and the third machine learning model were implemented on Python 3.10 with GPU-based Pytorch framework and the training was performed for 600 epochs with a batch size of 8 patients parallelized over 4 NVIDIA GTX 1080 Tis.

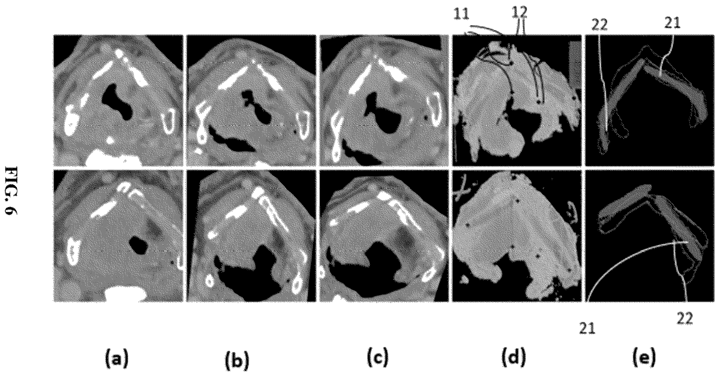

**Figure** 6 shows visual results obtained for two samples. Sub-image (a) represents the original 3D CT-scan image; Sub-figure (b) represents the warped 3D CT-scan image after step S3 (rigid registration); Sub- figure (c) represents the warped 3D-CT-scan image after step S4 (deformable registration); Sub- figure (d) represents the original histological image with landmarks from pathologist (11) and warped projected landmarks from radiologists (12) ; Sub- figure (e) represents an overlay of cartilage masks after registration of the histological image (21) and CT-scan (22); It can be observed that the method 100 succeeds in mapping the CT scan imaging modality and the histology imaging, on cartilage as well as on free soft tissue (cf. figures 6(c)(d)(e)).

Then , performance of the method 100 for generating a registered image (CT scan 3D image finely registered onto transformed histological 2D images) has been quantitatively assessed against three prior art methods, namely the registration with the VoxelMorph 3D architecture, the VoxelMorph 2/3D architecture and with an in-house prior registration method, by using three metrics quantifying the similarity between both images, namely the Dice coefficient, the Hausdorff Distance between cartilages and the average distance between characteristics landmarks disposed before registration, like trachea edges.

The results are provided below:

| Method | Dice (%) | Hausdorff (mm) | Landmark Distance error (mm) | Inference Runtime (seconds) |
|---|---|---|---|---|
| VoxelMorph 2D | 68.4±0.6 | 8.53±0.32 | 6.71±0.16 | 1.3 |
| VoxelMorph 2/3D | 71.9±1.7 | 7.19±0.24 | 5.99±0.22 | 1.4 |
| In-house prior method | 76.3±1.4 | 6.88±0.28 | 4.98±0.15 | 2.1 |
| Method 100 (no init) | 77.9±1.9 | 6.91±0.19 | 4.73±0.31 | 2.1 |
| Method 100 (no regu) | 85.1±0.8 | 4.23±0.27 | 3.71±0.19 | 2.8 |
| **Method 100** | **86.9±1.3** | **3.81±0.20** | **3.28±0.16** | **2.9** |

In the table above, "Method 100 (no init)" represents the method 100 where step S3 is not carried out, and "Method 100 (no regu)" represents the method 100 where no regulation parameters are used in step S4.

It can be observed that the method 100 outperforms all other methods, proving the necessity of a singular approach to handle the specific case of histology. Concerning the runtime (all methods are trained on GPU), with a 3-step cascade for initialization, the inference when using the method 100 remains in a similar time scale to other methods.

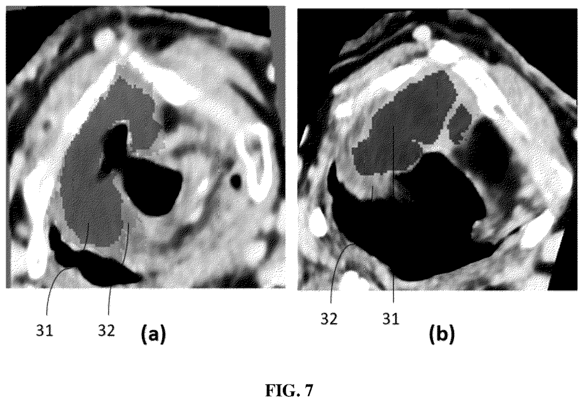

**Figures 7a and 7b** show two samples with an overlay of tumor masks from both the histology modality and the CT scan modality. More precisely, the tumor contours between WSI (31) and registered CT scan (32) are overlaid. Indeed, one clinical endpoint of the method 100 is to compare a gross tumor volume (GTV) delineated on CT-scan images with WSI-based gold-standard tumor extent after registration. A tumor variability, that is, a difference in the tumor estimation between both imaging modalities, can be observed.

Statistical data relating to the results corresponding to **Figures 7a and 7b** are shown below, namely the tumor variability estimation with the Hausdorff distance metrics, the Dice coefficient metrics, a sensitivity index and an inclusion index are reported below. The sensitivity index is representative of the proportion of histological tumor contained within the CTscan contour 32. The inclusion index of positive predictive value (PPV) is the proportion of gross tumor value that is pathological.

| Metric | Tumor variability |
|---|---|
| Hausdorff | 14.2±1.8 |
| Dice | 0.53±0.08 |
| Sensitivity | 0.86±0.09 |
| Inclusion /PPV | 0.66±0.12 |

One key point that can be noticed is that the gross tumor volume (GTV) delineated on CT overestimates the true tumor extent of around 31%, with some serious differences as demonstrated by the Hausdorff Distance or the low inclusion index. However, the GTV does not always encompass the tumor as the sensitivity index is not perfect. The typical error cases are the inclusion of cartilage, the consideration of edema, and the oversight of isolated tumor blocks. It leads to a very low Dice Coefficient, which highlights the limitations and variability of radiology-based (i.e. CT-scan radiology here) examinations, but also to increased toxicity (by a cubic factor), for example in radiation therapy. Therefore, the method 100 allows avoiding those current systematic errors in current clinical practice and allows for a better understanding of tumor environments.

A person skilled in the art will readily appreciate that various parameters disclosed in the description may be modified and that various embodiments disclosed may be combined without departing from the scope of the invention. Of course, the present invention is not limited to the embodiments described above as examples. It can be extended to other variants.

**Claims**

1. A computer-implemented method (100) for generating a registered image ($B_{reg}$) based on at least one first image of an object compliant with a source imaging modality and on at least one second image of said object compliant with a target imaging modality, the method comprising:

   - receiving said at least one first image ($a_{Zi}$) and said at least one second image (B),
   - transforming said at least one first image ($a_{Zi}$) into at least one first transformed image ($sb_{Zi}$), so that said at least one first transformed image ($sb_{Zi}$) is compliant with said target imaging modality, by using a first machine learning model,
   - implementing a second machine learning model receiving as inputs said at least one second image and at least one first transformed image ($sb_{Zi}$), so as to obtain at least one first registered image (B'),
   - implementing a third machine learning model, said third machine learning model receiving as inputs said at least one first transformed image ($sb_{Zi}$) and said at least one first registered image (B'), so as to obtain said registered image ($B_{reg}$), wherein said registered image ($B_{reg}$) is registered on said at least one first transformed image ($sb_{Zi}$).

2. The method according to any of the preceding claims, wherein said object is a human body part.

3. The method according to claim **1** or **2**, wherein the at least one first image ($a_{Zi}$) comprises at least one 2D image and wherein the at least one second image comprises one 3D image, said at least one 3D image (B) comprising a plurality of N 2D sections ($b_i$) of said object.

4. The method according to claim **1** or **2**, wherein the at least one first image comprises at least one 3D image (B) comprising a plurality of N 2D sections ($b_i$) of said object and wherein the at least one second image comprises at least one 2D image.

5. The method according to claim **3**, wherein implementing said second machine learning model comprises iterations, each iteration among said iterations comprising:

   - i) registering said at least one second image onto said at least one first transformed image ($sb_{Zi}$) by applying a current global rigid transform to said at least one second image, to obtain at least one current second transformed image, said current second transformed image comprising N current 2D transformed sections of said object,
   - ii) matching each first transformed image ($sb_{Zi}$) among the at least one first transformed images ($sB_{Zi}$) to one corresponding current 2D transformed section among the N current second transformed sections so as to meet a similarity criterion, said similarity criterion being evaluated on the basis of a similarity measure estimated between each first transformed image ($sb_{Zi}$) and one corresponding current 2D transformed section.

6. The method according to claim **5**, further comprising, preliminarily to implementing the second machine learning model,: :

   - obtaining at least one first segmentation mask ($M_A$) corresponding to structures of interest in said at least one first image ($a_{Zi}$),
   - obtaining at least one second segmentation mask ($M_B$) corresponding to said structures of interest in said plurality of N 2D sections ($b_i$),

and wherein step i) of each iteration comprises implementing said second machine learning model receiving as additional inputs said at least one first segmentation mask ($M_A$) and said at least one second segmentation mask ($M_B$), so as to obtain said current global rigid transform.

7. The method according to claim **6** and claim **2,** wherein the structures of interest are stiff regions such as bones, cartilage, or tendons.

8. The method according to any of claims **5** or **6,** wherein step ii) of each iteration comprises updating, for each first transformed image ($sb_{Zi}$), a coordinate along a common axis of said plurality of N current 2D transformed sections, by maximizing the similarity measure.

9. The method according to any of the preceding claims, wherein the first machine learning model is a cycle generative adversarial network, GAN, said first machine learning model being configured to generate, from an input source image compliant with said source imaging modality, a simulated image compliant with said target imaging modality respectively associated with said input source image.

10. The method according to any of the preceding claims and claim **3,** wherein:

    - said at least one first transformed image ($sb_{Zi}$) corresponds to a first 3D representation ($sB_{complete}$) of said object,
    - the at least one first registered image (B') comprises one 3D first registered image with voxels and
    - the third machine learning model outputs, for each voxel, displacement information corresponding to a displacement between said one 3D first registered image and said first 3D representation ($sB_{complete}$).

11. The method according to claim **10** and to claim **6** or **7,** wherein, for each voxel, the displacement information takes into account a regularization parameter that is a function of said voxel position with respect to said structures of interest.

12. The method according to any of the preceding claims and claim **3,** wherein the source imaging modality is histopathology, whole slide imaging, or echography.

13. The method according to any of the preceding claims and claim **3,** wherein the target imaging source modality is computed tomography imaging or magnetic resonance imaging.

14. A device comprising a processor configured to carry out a method according to any one of claims **1** to **13.**

15. A computer program product comprising instructions, which, when the program is executed by a computer, cause the computer to carry out the method of any one of claims **1** to **13.**

**FIG. 1**

Receiving P 2D images and a 3D image — S1

Transforming the P 2D images into first transformed images $sb_{zi}$ — S2

100

Obtaining a first registered image B' — S3

Obtaining a finely registered image $B_{reg}$ — S4

**FIG. 2**

**FIG. 3**

FIG. 4

FIG. 5

FIG. 6

**FIG. 7**

Europäisches
Patentamt

European
Patent Office

Office européen
des brevets

# EUROPEAN SEARCH REPORT

**Application Number**

EP 23 16 7630

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MALTE HOFFMANN ET AL: "Anatomy-aware and acquisition-agnostic joint registration with SynthMorph", ARXIV.ORG, CORNELL UNIVERSITY LIBRARY, 201 OLIN LIBRARY CORNELL UNIVERSITY ITHACA, NY 14853, 26 January 2023 (2023-01-26), XP091421868, | 1-4,9, 10,12-15 | INV. G06T7/30 |
| A | * Fig. 4. Sec. 3.2.3, Sec. 4.1, Sec. 4.5. * | 5-8,11 | |
| X | CASAMITJANA ADRIÀ ET AL: "Synth-by-Reg (SbR): Contrastive Learning for Synthesis-Based Registration of Paired Images", 21 September 2021 (2021-09-21), TOPICS IN CRYPTOLOGY - CT-RSA 2020 : THE CRYPTOGRAPHERS' TRACK AT THE RSA CONFERENCE 2020, SAN FRANCISCO, CA, USA, FEBRUARY 24-28, 2020, CORNELL UNIVERSITY LIBRARY, 201 OLIN LIBRARY CORNELL UNIVERSITY ITHACA, NY 14853, PAGE(S) 44 - 54, XP047610408, [retrieved on 2021-09-21] * Fig. 1 and Sec. 2. * | 1,2,9, 12-15 | |
| X | US 10 346 974 B2 (TOSHIBA MEDICAL SYS CORP [JP]) 9 July 2019 (2019-07-09) * col. 5, l. 15-26, 32-41. Fig. 7, stages 72, 76 and column 11 l. 8-18. * | 1-4,9, 12-15 | |

TECHNICAL FIELDS SEARCHED (IPC)

G06T

-/--

The present search report has been drawn up for all claims

| Place of search | Date of completion of the search | Examiner |
|---|---|---|
| Munich | 5 October 2023 | Nicolau, Stephane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LEROY AMAURY ET AL: "End-to-End Multi-Slice-to-Volume Concurrent Registration and Multimodal Generation", 17 September 2022 (2022-09-17), 20220917, PAGE(S) 152 – 162, XP047633809, [retrieved on 2022-09-17] * Fig. 1. Sec. 2.1 and 2.2 .Sec. 3.1. * ----- | 1-4,9, 12-15 | |

|  |  |  | TECHNICAL FIELDS SEARCHED (IPC) |
|---|---|---|---|

The present search report has been drawn up for all claims

| Place of search | Date of completion of the search | Examiner |
|---|---|---|
| Munich | 5 October 2023 | Nicolau, Stephane |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

# EP 4 446 988 A1

## ANNEX TO THE EUROPEAN SEARCH REPORT
## ON EUROPEAN PATENT APPLICATION NO.

EP 23 16 7630

This annex lists the patent family members relating to the patent documents cited in the above-mentioned European search report.
The members are as contained in the European Patent Office EDP file on
The European Patent Office is in no way liable for these particulars which are merely given for the purpose of information.

05-10-2023

| Patent document cited in search report | | Publication date | Patent family member(s) | | Publication date |
|---|---|---|---|---|---|
| US 10346974 | B2 | 09-07-2019 | JP | 7246866 B2 | 28-03-2023 |
| | | | JP | 2018192264 A | 06-12-2018 |
| | | | US | 2018336677 A1 | 22-11-2018 |

EPO FORM P0459

For more details about this annex : see Official Journal of the European Patent Office, No. 12/82